Europäisches Patentamt

European Patent Office

Office européen des brevets

(19)

(11) Numéro de publication : **0 524 266 B1**

(12) **FASCICULE DE BREVET EUROPEEN**

(45) Date de publication du fascicule du brevet :
03.11.93 Bulletin 93/44

(51) Int. Cl.[5] : **A61K 7/48, A61K 35/78**

(21) Numéro de dépôt : **91908696.7**

(22) Date de dépôt : **09.04.91**

(86) Numéro de dépôt international :
**PCT/FR91/00289**

(87) Numéro de publication internationale :
**WO 91/16036 31.10.91 Gazette 91/25**

(54) **COMPOSITIONS COSMETIQUES CONTENANT UN EXTRAIT D'UNE ZYGOPHYLLACEE APPARTENANT AU GENRE LARREA.**

(30) Priorité : **12.04.90 FR 9004737**

(43) Date de publication de la demande :
**27.01.93 Bulletin 93/04**

(45) Mention de la délivrance du brevet :
**03.11.93 Bulletin 93/44**

(84) Etats contractants désignés :
**AT BE CH DE DK ES FR GB GR IT LI LU NL SE**

(56) Documents cités :
**WO-A-87/06833
International Journal of Dermatology, Vol. 19, No. 4, May 1980, M. El-Saad El-Rifaie: "Peganum harmala: its use in certain derma- toses", pages 221-222
Chemical Abstracts, Vol. 101, No. 7, 13 August 1984 (Colombus, Ohio, US) K. Masayuki et al.: "Interaction in the antibacterial activity of fla- vonoids from Sophora japonica L. to Propio- nicbacterium", see page 331, ref. 51606p, & Yakugaku Zasshi, 1984, 104(4), pages 340-6
Patents Abstracts of Japan, Vol. 8, No. 134 (C-230)(1571) 21 JUNE 1984, & JP-A-59 44 313 (Yakult Honsha K.K.) 12 March 1984**

(73) Titulaire : **BONNE, CLAUDE
316, avenue d'Occitanie
F-34000 Montpellier (FR)**
Titulaire : **SINCHOLLE, Daniel
343, avenue de la Trémoulette,
St-Clément-la-Rivière
F-34270 St-Mathieu-de-Tréviers (FR)**
Titulaire : **DIOT, MICHEL
2, allée des Dimanches
F-78430 Louveciennes (FR)**

(72) Inventeur : **BONNE, CLAUDE
316, avenue d'Occitanie
F-34000 Montpellier (FR)**
Inventeur : **SINCHOLLE, Daniel
343, avenue de la Trémoulette,
St-Clément-la-Rivière
F-34270 St-Mathieu-de-Tréviers (FR)**
Inventeur : **DIOT, MICHEL
2, allée des Dimanches
F-78430 Louveciennes (FR)**

(74) Mandataire : **Le Guen, Gérard et al
CABINET LAVOIX 2, place d'Estienne d'Orves
F-75441 Paris Cédex 09 (FR)**

EP 0 524 266 B1

## Description

La présente invention concerne une composition cosmétique s'opposant notamment à la formation des papulo-pustules acnéiques.

La présente invention concerne plus particuliérement une composition cosmétique contenant à titre de principe actif un extrait de feuilles et tiges d'au moins une zygophyllacée du genre Larrea.

Il est rapporté dans la littérature que des extraits de Larrea étaient utilisés par les Indiens d'Amérique pour le traitement du cancer ainsi que de la tuberculose et des infections rénales. Ces indications empiriques pourraient être fondées, car des travaux scientifiques récents ont en effet montré que des extraits de plantes appartenant à la famille des zygophyllacées présentaient une activité antitumorale (KATO R. et al. Chemical Abstracts, vol. 100, 1984, N° 29365s) activité antibactérienne est mise à profit dans diverses préparations commerciales destinées à la désinfection de la peau (LEWIS W.H. in medical botany plants affecting man's health. John Wiley and Sons, New York, 1977, p. 363, 366).

Les demandeurs ont découvert que l'extrait de Larrea présente, indépendamment des effets pharmacologiques connus, une remarquable activité inhibitrice de la migration leucocytaire et que des compositions topiques contenant de 0,01 à 0,1 % de polyphénols provenant de l'extrait, peuvent s'opposer à la pustulation des lésions dans un modèle d'acné expérimental.

L'acné vulgaire est une dermatose folliculaire qui effecte les adolescents et les adultes jeunes avec une grande fréquence. Elle atteint certaines unités pilo-sébacées de la face et du tronc, appelées follicules sebacées. Cette affection se manifeste par des lésions polymorphes ; la lésion primaire est le comédon à partir duquel peuvent se développer des lésions inflammatoires et cicatricielles. L'affection peut se réduire à une séquence de deux étapes.

La première consiste en la formation d'un comédon : sous diverses influences et principalement celle des hormones androgènes, la glande sebacée secrète les sébum dont les triglycérides sont hydrolysés grâce à des lipases produies par la flore folliculaire. Les acides gras libres formés et d'autres métabolites produits par la flore stimulent alors la formation de cellules kératinisées cohésives par l'infundibulum folliculaire, constituant ainsi un comédon clos.

La seconde étape est caractérisée par l'induction d'une papulo-pustule inflammatoire : le comédon clos peut soit se transformer en comédon ouvert, soit provoquer la rupture du sac folliculaire et des lésions inflammatoires. Plusieurs facteurs concourent à la rupture de l'épithélium parmi lesquels : la compression mécanique de la paroi épithéliale par le comédon et la libération d'enzymes lytiques par la flore folliculaire. Le passage du contenu comédonien dans le derme adjacent provoque l'attraction de leucocytes qui, entretenant à leur tour l'inflammation, créent la papulo-pustule acnéique.

L'extrait de Larrea, par son activité inhibitrice de la migration leucocytaire, permet d'empêcher l'évolution des comédons en de disgracieuses papulo-pustules.

La présente invention a en conséquence pour objet une composition cosmétique permettant notamment de s'opposer à la transformation des comédons en papulo-pustules acnéiques ainsi qu'à la formation de rougeurs cutanées, caractérisée en ce qu'elle contient un extrait qui peut être obtenu à partir de feuilles et tiges d'au moins une zygophyllacée du genre Larrea, à des concentrations de 0,01 à 0,10 % exprimées en polyphénols dans une base cosmétique appropriée.

La composition cosmétique selon la présente invention peut également être utilisée contre les irritations cutanées mineures telles que celles provoquées par des piqûres d'insectes et des brûlures superficielles.

La présente invention a également pour objet un procédé pour s'opposer à la formation de papulo-pustules acnéiques, caractérisé en ce que l'on applique sur la peau un extrait qui peut être obtenu à partir de feuilles et tiges d'au moins une zygophyllacée appartenant au genre Larrea, à une concentration de 0,01 à 0,10 % exprimée en polyphénols dans une base cosmétique appropriée.

La présente invention a également pour objet un procédé de préparation d'une composition selon l'invention, caractérise en ce que l'on incorpore un extrait qui peut être obtenu à partir de feuilles et tiges d'au moins une zygophyllacée appartenant au genre Larrea, à une concentration de 0,01 à 0,10 % exprimée en polyphénols, dans une base cosmétique appropriée.

Dans la présente invention, on peut utiliser notamment l'une des trois espèces de Larrea ci-après :
Larrea divaricata Cav.
Larrea cuneifolia Cav.
Larrea tridentata Coult.

L'extrait des feuilles et tiges de Larrca peut être obtenu par macération des organes végétaux pulvérisés de Larrea dans du propylène glycol, notamment à raison de 3 à 10 parties en poids de propylène glycol par partie d'organes végétaux, en milieu alcalin, pendant 24 à 48 h à la température ambiante (15 à 25° C). Après filtration l'extrait a une teneur en polyphénol de 0,5 à 5 %.

2

A titre d'exemple, on soumet la plante sèche à une opération de pulvérisation de façon à obtenir une poudre telle que le refus au tamis d'ouverture de maille de 1 mm soit inférieure à 5 %.

A 1 kg de poudre on ajoute 1 litre d'hydroxyde de sodium 0,1 N et 9 litres de propylène glycol de qualité pharmaceutique.

On place sous agitation mécanique lente (150 à 200 tours/minute) pendant 24 heures. On filtre de façon à obtenir un extrait liquide parfaitement limpide. On obtient ainsi 7 litres d'extrait.

On contrôle la teneur en polyphénols de l'extrait par dosage à l'aide du réactif d'Arnow. La teneur en polyphénol ne doit pas être inférieure à 0,5 %, exprimé en acide nor-dihydroguaiarétique.

Ci-après on désignera par "Extrait de Larrea" un tel extrait obtenu par macération avec du propylène glycol.

Les études suivantes mettent en évidence l'activité anti-acnéique du principe actif utilisé dans la présente invention.

Activité inhibitrice de la migration des leucocytes

La migration des leucocytes neutrophiles vers un site sous-cutané a été déclenché chez le rat par injection sous-cutanée de 5 ml de carboxyméthycellulose à 1 % dans une solution stérile isotonique de chlorure de sodium.

Des rats (témoins) ont reçu simultanément une injection au même point de 0,1 ml de propylène glycol et des rats (traités) ont reçu 0,1 ml d'Extrait de Larrea contenant 0,5 % de polyphénols. Six heures après ces traitements, les exsudats inflammatoires ont été ponctionnés et les leucocytes ont été dénombrés dans une cellule de Thomas après coloration.

Les résultats sont rapportés dans le Tableau I. Ces résultats montrent que l'Extrait de Larrea inhibe la migration des leucocytes vers le site inflammatoire sous-cutané.

## TABLEAU I
### Activité inhibitrice de la migration des leucocytes

| Extrait de Larrea | Concentration leucocytaire $\times\ 10^6/mm^3$ |
|---|---|
| 0 | $40 \pm 2$ |
| + | $21 \pm 1,5$ |

2 - Inhibition de la pustulation de lésions acnéiques expérimentales

Des lésions acnéiques expérimentales ont été induites sur la peau dorsale des lapins. Après rasage, des scarifications au scalpel ont été pratiquées et un homogénat d'épiderme humain a été appliqué sur les incisions cutanées (l'homogénat d'épiderme, modèle de matériel comédonien, a été préparé à partir de peau prélevée au cours d'interventions chirurgicales pour mammoplastie; l'épiderme a été collecté au cryomicrotome et homogénéisé dans un tampon phosphate isotonique).

Des scarifications ont en outre été traitées localement par 0,05 ml de propylène glycol ou d'Extrait de Larrea contenant 0,5 % de polyphénols, utilisé pur ou dilué. Après 24 heures, les lésions cutanées ont été examinées par deux observaturs, "en aveugle", et la réaction a été évaluée d'après l'aspect clinique de la lésion selon une échelle de 1 + à 4 +.

les résultats sont rapportés dans le tableau II. Ces résultats montrent que l'Extrait de Larrea s'oppose à la réaction inflammatoire induite par le matériel comédomimétique.

## TABLEAU II

### Activité inhibitrice de la migration des leucocytes

| Extrait de Larrea | Matériel comédo-mimétique | Réaction papulo-pustuleuse |
|---|---|---|
| O | O | + |
| O | + | ++++ |
| Dilution au 1/10 | + | ++ |
| Non dilué | + | + |

Les compositions cosmétiques selon l'invention peuvent en outre contenir éventuellement toutes les substances connues ayant des propriétés bienfaisantes sur les téguments et particulièrement pour leur activité cosmétique dans les soins des peaux à tendance acnéique. Elles peuvent contenir des antiseptiques, des comédolytiques, des antiséborrhéiques, des parfums, des conservateurs et des colorants.

Les compositions cosmétiques selon l'invention peuvent se présenter sous toutes les formes utilisées en cosmétologie, crème ou gel en pots ou en tubes, lait, lotion en flacon de verre ou de plastique, en ampoules ou en flacons doseurs.

Pour chaque forme particulière, on a recours à des excipients appropriés. Ces excipients doivent avoir toutes les qualités habituellement demandées. A titre d'exemples, on peut citer : le propylène glycol, la glycérine, l'alcool cétylique, les polyols, les phospholipides sous forme de liposomes ou non, les huiles végétales, animales, minérales, les mouillants, les épaississants, stabilisants et émulsionnants couramment utilisés.

Les différentes formes cosmétiques mentionnées ci-dessus sont obtenues selon les méthodes utilisées dans ce domaine.

On donnera ci-après des exemples de compositions cosmétiques (en parties de poids) :

1 - Lotion hydro-alcoolique

. Extrait de Larrea à 0,5 % de polyphénols     10
. Alcool cétylique     0,5
. Acide stéarique     5
. Glycérol     2
. Alginate de Na     0,3
. Triéthanolamine     0,5
. Alcool à 95°     25
. Eau q.s.p.     100

2 - Lait H/E

. Extrait de Larrea à 0,5 % de polyphénols     5
. Polyéthoxyétherphosphate d'alcool oléique     2
. Alcool cétylique     0,5
. Lanoline anhydre     0,5
. Silicone fluide     1
. Huile de germe de blé     2
. Huile de cade     2
. Acide stéarique     3
. Triéthanolamine     0,5
. Silicate de magnésium et d'aluminium     0,5
. Composition aromatique     q.s.
. Conservateurs     q.s.

. Eau q.s.p. 100

3 - Hydrogel alcoolique

. Extrait de Larrea à 0,5 % de polyphénols 10
. Principes antiseptiques 0,5
. Triéthylamine 1,5
. Carbopol 940 1,5
. Alcool q.s.p. 100

4 - Hydrogel glycérine

. Extrait de Larrea à 0,5 % de polyphénols 7
. Alginate sodique 8
. Glycérol 25
. Solution de borax (15 %) dans le glycérol 20
. Thymol 2
. Eau q.s.p. 100

5 - Crème H/E

. Extrait de Larrea à 0,5 % de polyphénols 10
. Huile de germe de blé 7
. Stéarate de glycérol 5
. Propylène de glycol 3
. Triéthanolamine 0,5
. Lécithine de soja 1
. Conservateurs q.s.
. Eau q.s.p. 100

6 - Crème E/H

. Extrait de Larrea à 0,5 % de polyphénols 8
. Acide salicytique 0,5
. Silicate d'aluminium 2
. Oxyde de titane 2
. Cire blanche 20
. Huile de paraffine 60
. Eau q.s.p. 100

**Revendications**

1. Composition cosmétique permettant notamment de s'opposer à la formation de papulo-pustules acnéiques et de rougeurs cutanées, caractérisée en ce qu'elle contient un extrait qui peut être obtenu à partir de feuilles et tiges d'au moins une zygophyllacée appartenant au genre Larrea, à une concentration de 0,01 à 0,10 % exprimée en polyphénols, dans une base cosmétique appropriée.

2. Composition selon la revendication 1, caractérisée en ce que l'extrait est un extrait qui peut être obtenu par macération des feuilles et des tiges de Larrea dans du propylène glycol.

3. Utilisation d'un extrait des feuilles et tiges de Larrea pour la fabrication d'une composition cosmétique s'opposant à la formation de papulo-pustules acnéiques, ayant une teneur en extrait de 0,01 à 0,10% exprimée en polyphénols.

4. Procédé cosmétique pour s'opposer à la formation de papulo-pustules acnéiques, caractérisé en ce que l'on applique sur la peau un extrait qui peut être obtenu à partir de feuilles et de tiges d'au moins une zygophyllacée appartenant au genre Larrea, à une concentration de 0,01 à 0,10 % exprimée en polyphénols,

5

dans une base cosmétique appropriée.

5. Procédé selon la revendication 4, caractérisé en ce que l'extrait est un extrait qui peut être obtenu par macération des feuilles et des tiges de Larrea dans du propylène glycol.

6. Procédé de préparation d'une composition selon la revendication 1, caractérisé en ce que l'on incorpore un extrait qui peut être obtenu à partir de feuilles et tiges d'au moins une zygophyllacée appartenant au genre Larrea, à une concentration de 0,01 à 0,10 % exprimée en polyphénols, dans une base cosmétique appropriée.

7. Procédé selon la revendication 6, caractérisé en ce que l'extrait est un extrait obtenu par macération des feuilles et des tiges de Larrea dans du propylène glycol.

## Patentansprüche

1. Kosmetische Zubereitung insbesondere für die Bekämpfung der Entstehung von aknebedingten Papulopugteln und Hautrötungen,
**dadurch gekennzeichnet, daß** sie einen aus den Blättern und Stengeln von wenigstens einem Zygophyllaceae der Klasse Larrea gewonnenen Extrakt in einer Konzentration von 0,01 bis 0,10 %, ausgedrückt in Phenolen, in einer geeigneten kosmetischen Basissubstanz enthält.

2. Kosmetische Zubereitung nach Anspruch 1,
**dadurch gekennzeichnet, daß** dieser Extrakt ein Extrakt ist, der durch Mazeration der Blätter und Stiele von Larrea in Propylenglykol gewonnen wird.

3. Verwendung eines aus den Blättern und Stielen von Larrea gewonnenen Extraktes zur Herstellung einer kosmetischen Zubereitung für die Bekämpfung der Entstehung von aknebedingten Papulopusteln in einer Konzentration von 0,01 bis 0,10 %, ausgedrückt in Polyphenolen.

4. Kosmetisches Verfahren für die Bekämpfung der Entstehung von aknebedingten Papulopusteln,
**dadurch gekennzeichnet, daß** man auf die Haut einen Extrakt aufträgt, welcher aus den Blättern und Stielen wenigstens einer Zygophyllaceae der Klasse Larrea gewonnen wird, und der in einer Konzentration von 0,01 bis 0,10 %, ausgedrückt in Polyphenolen, in einer geeigneten kosmetischen Basissubstanz enthalten ist.

5. Verfahren nach Anspruch 4,
**dadurch gekennzeichnet, daß** dieser Extrakt ein Extrakt ist, weicher durch Mazeration von Blättern und Stielen von Larrea in Propylenglykol gewonnen werden kann.

6. Verfahren zur Herstellung einer kosmetischen Zubereitung nach Anspruch 1,
**dadurch gekennzeichnet, daß** ein Extrakt eingesetzt wird, welcher aus den Blättern und Stielen wenigstens einer Zygophyllaceae der Klasse Larrea gewonnen wird, und der in einer Konzentration von 0,01 bis 0,10 %, ausgedrückt in Polyphenolen, in einer geeigneten kosmetischen Basissubstanz enthalten ist.

7. verfahren nach Anspruch 6,
**dadurch gekennzeichnet, daß** dieser Extrakt ein Extrakt ist, welcher durch Mazeration von Blättern und Stielen von Larrea in Propylenglykol gewonnen wird.

## Claims

1. A cosmetic composition, particularly to combat the formation of papulae and pustules characteristic of acne, and the formation of cutaneous red patches, **characterised in that** it comprises an extract which can be obtained from leaves and stems of at least one member of Zygophyllaceae belonging to the genus Larrea, at a concentration of 0.01 to 0.1% expressed in terms of polyphenols, in an appropriate cosmetic base.

2. A composition in accordance with claim 1, **characterised in that** the extract is one which can be obtained by maceration of leaves and stems of Larrea in propylene glycol.

3. Use of an extract of leaves and stems of Larrea for the production of a cosmetic composition having an extract content of 0.01 to 0.1% expressed in terms of polyphenols, to combat the formation of papulae and pustules characteristic of acne.

4. A cosmetic process to combat the formation of papulae and pustules characteristic of acne, **characterised in that** an extract is applied to the skin, which extract can be obtained from leaves and stems of at least one member of Zygophyllaceae belonging to the genus Larrea, at a concentration of 0.01 to 0.1% expressed in terms of polyphenols, in an appropriate cosmetic base.

5. A process in accordance with claim 4, **characterised in that** the extract is one which can be obtained by maceration of leaves and stems of Larrea in propylene glycol.

6. A process for preparing a composition in accordance with claim 1, **characterised in that** an extract is included, which can be obtained from leaves and stems of at least one member of Zygophyllaceae belonging to the genus Larrea, at a concentration of 0.01 to 0.1% expressed in terms of polyphenols, in an appropriate cosmetic base.

7. A process in accordance with claim 6, **characterised in that** the extract is one obtained by maceration of leaves and stems of Larrea in propylene glycol.